# EUROPEAN PATENT APPLICATION

(11) **EP 0 955 060 A1**
(43) Date of publication of application: **10.11.1999**
(21) Application number: 97928485.8
(22) Date of filing: 26.06.1997
(51) Int. Cl.: A61K 39/395, C07K 16/24, C12P 21/08

(54) **REMEDIES FOR ACUTE PULMONARY INJURIES DUE TO INDIRECT CAUSES CONTAINING ANTI-IL-8 ANTIBODY AS THE ACTIVE INGREDIENT**

(30) Priority: 26.06.1996 JP 20091896; 22.10.1996 JP 31537896
(71) Applicant: Chugai Seiyaku Kabushiki Kaisha, Tokyo 115 (JP)
(72) Inventor: MATSUSHIMA, Kouji, Matsudo-shi, Chiba 271 (JP); YOKOI, Kenji, Kanazawa-shi, Ishikawa 921 (JP)
(74) Representative: Wakerley, Helen Rachael
(86) International application number: JP9702221
(87) International publication number: WO9749426

(57) **Abstract**

A therapeutic agent for treatment of acute lung injury, especially acute respiratory distress syndrome and adult respiratory distress syndrome, resulting from indirect causes, especially the sepsis syndrome, severe nonthoracic trauma, hypertransfusion during emergency resuscitation, and an artificial cardiopulmonary bypass surgery, and a therapeutic agent for hypoxemia in acute lung injury, said agents comprising anti-IL-8 antibody as an active ingredient.

## Description

### Technical Field

The present invention relates to a therapeutic agent for acute lung injury resulting from indirect causes comprising anti-interleukin-8 (IL-8) antibody as an active ingredient. More specifically, the present invention relates to a therapeutic agent for acute respiratory distress syndrome and adult respiratory distress syndrome resulting from the sepsis syndrome and the like.

### Background Art

IL-8 is a protein that belongs to the C-X-C chemokine subfamily and was formerly designated as the monocyte-derived neutrophil chemotactic factor, the neutrophil attractant/activation protein-1, the neutrophil activating factor and the like. IL-8 is a factor that induces activation and migration of neutrophils, and is produced by inflammatory cytokines such as IL-1β and TNF-α (Koch, A.E. et al., J. Investig. Med. (1995) 43, 28-38; Larsen, C.G. et al., Immunology (1989) 68, 31-36), mitogens such as PMA and LPS (Yoshimura, T. et al, Proc. Natl. Acad. Sci. U.S.A. (1987) 84, 9233-9237), and heavy metals such as Cadmium (Horiguchi, H. et al., Lymphokine Cytokine Res. (1993) 12, 421-428) in a variety of cells. It is also known that human umbilical endothelial cells under a low oxygen condition express IL-8 (Karakurum, M. et al., J. Clin. Invest. (1994) 93, 1564-1570).

In order for IL-8 to exhibit its biological activity, it is necessary that IL-8 binds to IL-8 receptor and thereby stimulates the cells that are expressing IL-8 receptors. IL-8 receptors that transmit signals into the cell by binding to IL-8 have already been cloned and the amino acid sequences thereof have been elucidated. Human IL-8 receptors include those referred to as IL-8 receptor A (CXCR-1, α or 2) and those referred to as IL-8 receptor B (CXCR-2, β or 1) (Murphy, P. M. and Tiffany, H.L., Science (1991) 253, 1280-1283; Holmes, W.E. et al., Science (1991) 253, 1278-1280). Both are thought to have a structure that penetrates the cell membrane seven times, and both are associated with GTP-binding proteins in the cytoplasmic domain (Horuk, R., Trends Pharmacol. Sci. (1994) 15, 159-165), and transmit IL-8 signals into the cell. Therefore, inhibition of binding between IL-8 and IL-8 receptor enables the inhibition of biological activity of IL-8.

Acute lung injury is a definition proposed in 1994 by a European and American consensus conference held in 1992 which is composed of the American Thoracic Society and the European Society of Intensive Care Medicine (Bernard, G.R. et al., Am J. Respir. Crit. Care Med. (1994) 149, 818-824). The clinical picture of the disease follows a course of acute onset and exhibits hypoxemia and bilateral diffuse infiltrative shadow in the X-ray chest radiograph, and the disease is diagnosed when these clinical findings do not result from left atrial or pulmonary capillary high blood pressure.

As a specific measure of hypoxemia, acute lung injury is defined by a PaO₂/FIO₂ value of 300 mmHg or lower which is an index of the respiratory function. Among acute lung injuries, those having a more severe hypoxemia, i.e. specifically having a PaO₂/FIO₂ value of 200 mmHg or lower, are diagnosed as acute respiratory distress syndrome, which exhibits symptoms almost identical to those of adult respiratory distress syndrome. Since the pathological conditions of adult respiratory distress syndrome are seen not only in adults but also in children, it has been proposed that the name of adult respiratory distress syndrome be changed to acute respiratory distress syndrome.

Pathological conditions of acute respiratory distress syndrome or adult respiratory distress syndrome are pulmonary edema due to increased permeability based on injuries in the pulmonary microvascular endothelia, which is a severe respiratory failure with a death rate as high as about 60% (Matthay, M.A., Clin. Chest Med. (1990) 11, 575-580). Besides, many patients die within 14 days. Causes that induce acute respiratory distress syndrome or adult respiratory distress syndrome are broadly divided into the direct causes that occur in the lung itself and directly injure it and the indirect causes that occur systemically and indirectly injure the lung (Bernard, G.R. et al)., Am. J. Respir. Crit. Care Med. (1994) 149, 818-824).

The direct causes are classified into aspiration, diffuse pulmonary infection, near-drowning, inhalation of irritant gas, and lung contusion. The indirect causes are classified into the sepsis syndrome, severe nonthoracic trauma, hypertransfusion during emergency resuscitation, and an artificial cardiopulmonary bypass surgery. Among these causes, one that has a high incidence and bad prognosis as a cause of acute lung injury is the sepsis syndrome (Montgomery, A.B. et al., Am. Rev. Respir. Dis. (1985) 132, 485-489, Knaus, W.A. et al., Am. J. Respir. Crit. Care Med. (1994) 150, 311-317). The definition of the sepsis syndrome was not explicitly discussed in the meeting of the European and American consensus conference held in 1992, but has been generally defined by Bone, R.C. (Ann. Intern. Med. (1991) 114, 332-333). Also, in a proposal made by a consensus conference of the American College of Chest Physicians: ACCP) and the Society of Critical Care Medicine: SCCM) held in 1991, the definition of sepsis was established, specifying that it does not necessarily require clinical findings as an infection (Bone, R.C. et al., Chest (1992) 101, 1644-1655: Crit. Care Med. (1992) 20, 864-874). When the concepts of these diseases are compared, the sepsis syndrome is included into sepsis as specified by the ACCP/SCCM.

Indirect causes of acute lung injury, acute respiratory distress syndrome, and adult respiratory distress syndrome which are a subject of treatment of the present invention resulting from indirect causes may be either sepsis or the sepsis syndrome. Acute lung injury, acute respiratory distress syndrome, and adult respiratory distress syndrome are differentiated from sepsis or the sepsis syndrome in that the former diseases have concurrently both bilateral diffuse infiltrative shadow in the x-ray chest radiograph and a PaO₂/FIO₂ value of 300 mmHg or lower in their clinical findings.

Since acute respiratory distress syndrome and adult respiratory distress syndrome occur secondarily to various causative diseases, the mechanism of onset has not been fully elucidated and efforts to elucidate it are currently under way from various aspects. It is estimated that some stimulation induced by various causative diseases activates certain attacking factors, which initiates the process of lung injury.

Attacking factors are broadly divided into the humoral factors and the cellular factors: as the humoral factors, cytokines such as IL-1 (Suter, P.M. et al., Am. Rev. Respir. Dis. (1992) 145, 1016-1022), IL-6 (Meduri, G.U. et al., Chest (1995) 108, 1315-1325), IL-8 (Miller, E.J. et al., Am. Rev. Respir. Dis. (1992) 146, 427-432), and TNF-α (Marks, J.D. et al., Am. Rev. Respir. Dis. (1990) 141, 94-97), complements (Hammerschmidt, D.E. et al., Lancet (1980) 1 (8175), 947-949), proteolytic enzymes (Farjanel, J. et al., Am. Rev. Respir. Dis. (1993) 147, 1091-1099), arachidonic acid metabolites (Stephenson, A.H. et al., Am. Rev. Respir. Dis. (1988) 138, 714-719), PAF (Matsumoto., K. et al., Clin. Exp. Pharmacol. Physiol. (1992) 19, 509-515), activated oxygen (Left, J.A. et al., Am. Rev. Respir. Dis. (1992) 146, 985-989), and many other factors; and as the cellar factors, neutrophils (Weiland, J.E. et al., Am. Rev. Respir. Dis. (1986), 133, 218-225), and alveolar macrophages (Tran Van Nhieu, J. et al., Am. Rev. Respir. Dis. (1993) 147, 1585-1589) have been estimated to be attacking factors since they are detected in bronchoalveolar lavage or the peripheral blood and the like.

These attacking factors are considered to relate to each other forming intricate networks, which are believed to compound pathological conditions of the diseases and render the treatment of the diseases difficult. On the other hand, some of these attacking factors serve to be protective for the living body, i.e. to alleviate injuries or to act as the mechanism of repairing. Accordingly, it is not clear yet which attacking factors are to be targeted in order to establish a treatment regimen.

A causative disease most highly noted because of its high incidence is sepsis resulting from a gram negative microorganism which is one of the indirect causes; a well known stimulating agent in this case is endotoxin. In an acute lung injury model in which aspiration, one of the direct causes, was simulated by inhalation of hydrochloric acid, anti-IL-8 antibody was shown to be effective (Folkesson, H.G. et al., J. Clin. Invest. (1995) 107-116). It was not known at all, however, that anti-IL-8 antibody has a therapeutic effect for acute lung injury resulting from indirect causes such as the sepsis syndrome which occurs systemically and thereby injures the lung indirectly.

### Disclosure of the Invention

Improvement of vital prognosis can be expected by treating diseases resulting from these indirect causes. At present, therapeutic methods for these diseases depend on symptomatic therapies for life support, and do not provide treatments that inhibit lung injury itself and proceed with repairing. Furthermore, the effects of these symptomatic therapies are transient in most cases, and the death rate of adult respiratory distress syndrome is as high as about 60% even now (Matthay, M.A. et al., Clin. Chest Med. (1990) 11, 575-580).

Thus, it has been desired to develop a new drug as a therapeutic agent for acute lung injury. Steroids are now considered to be not so effective as had once been expected to be for prevention of onset of adult respiratory distress syndrome or after onset of adult respiratory distress syndrome (Kanazawa, Minoru, "Kyuukyuu Igaku (Emergency Medicine)" (1991) 15, 753-760).

Thus, it is an object of the present invention to provide a new therapeutic agent which resolves the above problems.

As a result of an intensive study to provide such a therapeutic agent, the applicants have found that an anti-IL-8 antibody can attain the desired object, and thereby have completed the present invention.

Thus, the present invention provides a therapeutic agent for acute lung injury resulting from indirect causes comprising anti-IL-8 antibody as an active ingredient.

The present invention also provides a therapeutic agent for acute respiratory distress syndrome resulting from indirect causes comprising anti-IL-8 antibody as an active ingredient.

The present invention also provides a therapeutic agent for adult respiratory distress syndrome resulting from indirect causes comprising anti-IL-8 antibody as an active ingredient.

The present invention also provides a therapeutic agent for acute lung injury resulting from the sepsis syndrome comprising anti-IL-8 antibody as an active ingredient.

The present invention also provides a therapeutic agent for acute respiratory distress syndrome resulting from the sepsis syndrome comprising anti-IL-8 antibody as an active ingredient.

The present invention also provides a therapeutic agent for adult respiratory distress syndrome resulting from the sepsis syndrome comprising anti-IL-8 antibody as an active ingredient.

The present invention also provides a therapeutic agent for acute lung injury resulting from severe nonthoracic trauma comprising anti-IL-8 antibody as an active ingredient.

The present invention also provides a therapeutic agent for acute respiratory distress syndrome resulting from severe nonthoracic trauma comprising anti-IL-8 antibody as an active ingredient.

The present invention also provides a therapeutic agent for adult respiratory distress syndrome resulting from severe nonthoracic trauma comprising anti-IL-8 antibody as an active ingredient.

The present invention also provides a therapeutic agent for acute lung injury resulting from hypertransfusion during emergency resuscitation comprising anti-IL-8 antibody as an active ingredient.

The present invention also provides a therapeutic agent for acute respiratory distress syndrome resulting from hypertransfusion during emergency resuscitation comprising anti-IL-8 antibody as an active ingredient.

The present invention also provides a therapeutic agent for adult respiratory distress syndrome resulting from hypertransfusion during emergency resuscitation comprising anti-IL-8 antibody as an active ingredient.

The present invention also provides a therapeutic agent for acute lung injury resulting from an artificial cardiopulmonary bypass surgery comprising anti-IL-8 antibody as an active ingredient.

The present invention also provides a therapeutic agent for acute respiratory distress syndrome resulting from an artificial cardiopulmonary bypass surgery comprising anti-IL-8 antibody as an active ingredient.

The present invention also provides a therapeutic agent for adult respiratory distress syndrome resulting from an artificial cardiopulmonary bypass surgery comprising anti-IL-8 antibody as an active ingredient.

The present invention also provides a therapeutic agent for acute lung injury resulting from the above indirect causes comprising anti-IL-8 monoclonal antibody as an active ingredient.

The present invention also provides a therapeutic agent for acute lung injury resulting from the above indirect causes comprising antibody against mammalian IL-8 as an active ingredient.

The present invention also provides a therapeutic agent for acute lung injury resulting from the above indirect causes comprising antibody against human IL-8 as an active ingredient.

The present invention also provides a therapeutic agent for acute lung injury resulting from the above indirect causes comprising WS-4 antibody as an active ingredient.

The present invention also provides a therapeutic agent for acute lung injury resulting from the above indirect causes comprising an anti-IL-8 antibody having the constant region of human antibody as an active ingredient.

The present invention also provides a therapeutic agent for acute lung injury resulting from the above indirect causes comprising a humanized anti-IL-8 antibody or a chimeric anti-IL-8 antibody as an active ingredient.

The present invention also provides a therapeutic agent for acute lung injury resulting from the above indirect causes comprising a humanized WS-4 antibody as an active ingredient.

The present invention also provides a therapeutic agent for hypoxemia in acute lung injury resulting from indirect causes comprising anti-IL-8 antibody as an active ingredient.

### Brief Explanation of Drawings

Fig. 1 is a graph showing a survival curve which compares the effect of WS-4 antibody on life lengthening with a control antibody in a model of acute lung injury resulting from indirect causes.
Fig. 2 is a graph showing a comparison of a time course of arterial blood gas values in the survival cases (N=5) of the WS-4 antibody administration group with that in the death cases (N=5) of the control antibody administration group in a model of acute lung injury resulting from indirect causes.
Fig. 3 is a graph showing a comparison of the effect of WS-4 antibody on the wet/dry weight ratio of the lung with that of the control antibody in a model of acute lung injury resulting from indirect causes.
Fig. 4 is a graph showing a comparison of the effect of WS-4 antibody on protein concentration in the bronchoalveolar lavage fluid with the control antibody in a model of acute lung injury resulting from indirect causes.

### Embodiment for Carrying Out the Invention

In accordance with the present invention, acute lung injury resulting from indirect causes is the subject of treatment. Acute lung injury follows a course of acute onset and exhibits hypoxemia and bilateral diffuse infiltrative shadow in the x-ray chest radiograph, and it is a disease in which these clinical findings are not considered to result from left atrial or pulmonary capillary high blood pressure. Some of the diseases result from direct causes that occur in the lung itself, and others result from indirect causes that occur systemically, but their mechanism of onset is quite different from each other.

Onset of acute lung injury resulting from direct causes is induced by invasion from the side leading to the outside of the body. For example, aspiration that is classified as a direct cause, in which when gastric contents are vomited and the vomit enters into the airway for some reason, gastric acid directly injures mucosa of the airway, epithelial cells of the airway, epithelial cells of the alveolar, and the like, thereby inducing acute lung injury. On the other hand, onset of acute lung injury resulting from indirect causes is induced by attacks from within the blood vessel. For example, sepsis that is classified as an indirect cause, in which endotoxins and lipopolysaccharides act as stimulating factors that induce injuries. Endotoxins elicit inflammatory reactions in the blood and thereby induce activation of the complement and the coagulation system and the production of cytokines, and the like. Combined with these secondary reactions, onset of the disease is induced.

### 1. Anti-IL-8 antibody

Anti-IL-8 antibodies for use in the present invention may be of any origin, any kind (monoclonal or polyclonal), and any form, as long as it has a therapeutic effect against acute lung injury, acute respiratory distress syndrome, and adult respiratory distress syndrome resulting from indirect causes such as the sepsis syndrome, severe nonthoracic trauma, hypertransfusion during emergency resuscitation, and an artificial cardiopulmonary bypass surgery.

Anti-IL-8 antibodies for use in the present invention can be obtained as polyclonal or monoclonal antibodies using a known method. As the anti-IL-8 antibodies for use in the present invention, monoclonal antibodies of, in particular, a mammalian origin, are preferred. Monoclonal antibodies of a mammalian origin include those produced by a hybridoma or a host which has been transformed with an expression vector containing genetically engineered antibody genes. Anti-IL-8 antibody for use in the present invention, via binding to IL-8, blocks the binding between IL-8 and IL-8 receptor expressed on neutrophils etc. and thereby inhibits signal transmission of IL-8, and is therefore an antibody which inhibits the biological activity of IL-8.

Examples of such antibodies include WS-4 antibody (Ko, Y. et al., J. Immunol. Methods (1992) 149, 227-235) and DM/C7 antibody (Mulligan, M.S. et al., J. Immunol. (1993) 150, 5585-5595), Pep-1 antibody and Pep-3 antibody (International Patent Application WO 92/04372), or 6G4.2.5 antibody and A5.12.14 antibody (International Patent Application WO 95/23865; Boylan, A.M. et al., J. Clin. Invest. (1992) 89, 1257-1267). Among them, WS-4 antibody is most preferred.

Incidentally, the hybridoma cell line which produces WS-4 antibody has been internationally deposited under the provisions of the Budapest Treaty as mouse hybridoma WS-4 on April 17, 1996 with the Fermentation Research Institute, Agency of Industrial Science and Technology, of 1-3, Higashi 1-chome, Tsukuba city, Ibaraki pref., Japan, as FERM BP-5507.

### 2. Antibody produced by hybridoma

Monoclonal antibodies can be basically constructed using a known procedure as described bellow. Thus, IL-8 is used as an immunizing antigen and is immunized in the conventional method of immunization. The immune cells thus obtained are fused with known parent cells in the conventional cell fusion process, and then screened by the conventional screening method to screen monoclonal antibody-producing cells.

Specifically, monoclonal antibodies may be obtained in the following manner.

For example, IL-8 used as the immunizing antibody for obtaining antibody can be obtained using the respective IL-8 gene/amino acid sequence as disclosed in Matsushima, K. et al., J. Exp. Med. (1988) 167, 1883-1893 for human IL-8, in Harada, A. et al., Int. Immunol. (1993) 5, 681-690 for rabbit IL-8, in Ishikawa, J. et al., Gene (1993) 131, 305-306 for canine IL-8, Seow, H.F. et al., Immunol. Cell Biol. (1994) 72, 398-405 for sheep IL-8, Villinger, F. et al, J. Immunol. (1995) 155, 3946-3954 for monkey IL-8, in Yoshimura, T. and Johnson, D.G., J. Immunol. (1993) 151, 6225-6236 for guinea pig IL-8, and in Goodman, R.B. et al., Biochemistry (1992) 31, 10483-10490 for porcine IL-8.

It is reported that human IL-8 is produced in a variety of cells and undergoes different processing at the N-terminal ends(Leonard, E.J. et al., Am. J. Respir. Cell. Mol. Biol. (1990) 2, 479-486). Though IL-8 that has 79, 77, 72, 71, 70 or 69 amino acid residues has been known so far, the number of amino acid residues is not limited in any way so long as the IL-8 can be used as the antigen for obtaining anti-IL-8 antibody which is used in the present invention.

The gene sequence of IL-8 is inserted into a known expression vector to transform an appropriate host cell. From the host cell or the culture supernatant thereof, the desired anti-IL-8 antibody is purified using a known method, and the IL-8 protein thus purified may be used as the immunization antigen.

Preferably mammals to be immunized with the immunization antigen are selected in consideration of their compatibility with the parent cells for use in cell fusion generally and they generally include, but are not limited to, rodents, logomorphas, and primates. As rodents, for example, mice, rats, hamsters, etc. are used. As logomorphas, for example, rabbits are used. As primates, for example, monkeys are used. As monkeys, catarrhines (Old-World monkeys) such as cynomolgi (crab-eating macaque), rhesus monkeys, sacred baboons, chimpanzees etc. are used.

Immunization of animals with an immunization antigen is carried out using a known method. A general method, for example, includes intraperitoneal or subcutaneous administration of an immunization antigen to the mammal. Specifically, an immunization antigen which was diluted and suspended in an appropriate amount of phosphate buffered saline (PBS) or physiological saline etc. is mixed with an appropriate amount of Freund's complete adjuvant. After being emulsified, it is preferably administered to a mammal for several times every 4 to 21 days. Additionally a suitable carrier may be used at the time of immunization of the immunization antigen.

After the immunization and confirmation of the increase in the desired antibody levels in the serum by a conventional method, the immune cells such as lymphatic cells or spleen cells are taken out from the mammal and are subjected to cell fusion, in which preferred immune cells are in particular the spleen cells.

The mammalian myeloma cells as the other parent cells which are subjected to cell fusion with the above-mentioned immune cells preferably include various known cell lines such as P3 (P3x63Ag8.653) (Kearney, J.F. et al., J. Immunol. (1979) 123, 1548-1550), P3x63Ag8U. 1 (Yelton, D.E. et al., Current Topics in Microbiology and Immunology (1978) 81, 1-7), NS-1 (Kohler, G. and Milstein, C., Eur. J. Immunol. (1976) 6, 511-519), MPC-11 (Margulies, D.H. et al., Cell (1976) 8, 405-415), SP2/0 (Shulman, M. et al., Nature (1978) 276, 269-270), F0 (de St. Groth, S.F. and Scheidegger, D., J. Immunol. Methods (1980) 35, 1-21 S194 (Trowbridge, I.S., J. Exp. Med. (1978) 148, 313-323), R210 (Galfre, G. et al., Nature (1979) 217, 131-133) and the like.

Cell fusion between the above immune cells and the myeloma cells may be essentially conducted in accordance with a known method such as is described in Milstein et al. (Galfre, G. and Milstein, C., Methods Enzymol. (1981) 73, 3-46) and the like.

More specifically, the above cell fusion is carried out in the conventional nutrient medium in the presence of, for example, a cell fusion accelerator. As the cell fusion accelerator, for example, polyethylene glycol (PEG), Sendai virus (HVJ) and the like may be used, and an assistant agent such as dimethyl sulfoxide etc. may be added as desired to enhance efficiency of the fusion.

The preferred ratio of the immune cells and the myeloma cells to be used is, for example, 1 to 10 times more immune cells than the myeloma cells. Examples of culture media to be used for the above cell fusion include RPMI 1640 medium and MEM culture medium suitable for the growth of the above myeloma cell lines, and a conventional culture medium used for this type of cell culture, and a serum supplement such as fetal calf serum (FCS) may be added.

In cell fusion, predetermined amounts of the above immune cells and the myeloma cells are mixed well in the above culture medium, to which a PEG solution previously heated to about 37 °C, for example a PEG solution with a mean molecular weight of 1000 to 6000, is added at a concentration of 30 to 60% (w/v) and mixed to obtain the desired fusion cells (hybridomas). Then by repeating the sequential addition of a suitable culture medium and centrifugation to remove the supernatant, cell fusion agents etc. which are undesirable for the growth of the hybridoma can be removed.

The hybridoma is selected by culturing in the conventional selection medium, for example, HAT culture medium (a culture medium containing hypoxanthine, aminopterin, and thymidine). Culturing in the HAT culture medium is continued generally for the period of time sufficient to effect killing of the cells other than the desired hybridoma (non-fusion cells), generally several days to several weeks. The conventional limiting dilution method is conducted in which the hybridomas producing the desired antibody are screened and monoclonally cloned.

In addition to obtaining the above hybridoma by immunizing a non-human animal with an antigen, it is also possible to immunize human lymphocytes in vitro with IL-8, and the resulting immunized lymphocytes are fused with a myeloma cell, for example U266, having the ability of dividing permanently to obtain a hybridoma that produces the desired human antibody having the activity of binding to IL-8 (Japanese Examined Patent Publication (Kokoku) 1-59878). Furthermore, a transgenic animal having a repertoire of human antibody genes is immunized with the antigen IL-8 to obtain anti-IL-8 antibody-producing cells. The cells are then fused with myeloma cells to obtain hybridomas that are used to obtain human antibody to IL-8 (see International Patent Application WO 92/03918, WO 93/12227, WO 94/02602, WO 94/25585, WO 96/33735 and WO 96/34096).

The monoclonal antibody-producing hybridomas thus constructed can be subcultured in the conventional culture medium, or can be stored for a prolonged period of time in liquid nitrogen.

In order to obtain monoclonal antibodies from said hybridoma, there can be mentioned a method in which said hybridoma is cultured in the conventional method and the antibodies are obtained as the supernatant, or a method in which the hybridoma is transplanted to and grown in a mammal compatible with said hybridoma and the antibodies are obtained as the ascites. The former method is suitable for obtaining high-purity antibodies, whereas the latter is suitable for a large scale production of antibodies.

In addition to using a hybridoma for antibody production, immune cells such as antibody-producing immunized lymphocytes which have been immortalized with an oncogene can be used.

### 3. Recombinant antibody

Monoclonal antibodies may be also obtained as a recombinant antibody which has been produced by the recombinant gene technology. For example, recombinant antibody can be obtained by cloning antibody genes from a hybridoma or an immune cell such as antibody-producing immunized lymphocytes, and then placed into a suitable vector, which is then introduced into a host to produce said antibody (see, for example, Borrebaeck, C.A.K. and Larrick, J.W., THERAPEUTIC MONOCLONAL ANTIBODIES, published in the United Kingdom by MACMILLAN PUBLISHERS LTD. 1990).

Specifically, mRNA encoding the variable region (V region) of anti-IL-8 antibody is isolated from the hybridoma producing anti-IL-8 antibody. The isolation of mRNA is conducted by preparing total RNA using, for example, a known method such as the guanidine ultracentrifugation method (Chirgwin, J.M. et al., Biochemistry (1979) 18, 5294-5299), the AGPC method (Chomczynski, P. and Sacchi, N., Anal. Biochem. (1987) 162, 156-159), and then mRNA is purified from the total RNA using an mRNA Purification kit (Pharmacia) or the like. Alternatively, mRNA can be directly prepared using the QuickPrep mRNA Purification Kit (Pharmacia).

cDNA of the V region of antibody may be synthesized from the mRNA thus obtained using a reverse transcriptase. cDNA may be synthesized using an AMV Reverse Transcriptase First-strand cDNA Synthesis Kit (Seikagaku Kogyo), and the like. Alternatively, for the synthesis and amplification of cDNA, the 5'-RACE method (Frohman, M.A. et al., Proc. Natl. Acad. Sci. U.S.A. (1988) 85, 8998-9002; Belyavsky, A. et al., Nucleic Acids Res. (1989) 17, 2919-2932) which employs a 5'-Ampli FINDER RACE Kit (Clontech) and polymerase chain reaction (PCR) may be used.

The desired DNA fragment is purified from the PCR product obtained and may be ligated to a vector DNA. Moreover, a recombinant vector is constructed therefrom and then is introduced into E. coli etc., a colony thereof is selected to prepare the desired recombinant vector. The nucleotide sequence of the desired recombinant DNA may be confirmed by a known method such as the dideoxy nucleotide chain termination method.

Once the DNA encoding the V region of the desired anti-IL-8 antibody has been obtained, it may be ligated to DNA encoding the constant region (C region) of the desired antibody, which is then placed into an expression vector. Alternatively, DNA encoding the V region of the antibody may be placed into an expression vector which already contains DNA encoding the C region of the antibody. The antibody C region may be derived from the same animal species as that of the V region, or from a different animal species from that of the V region.

In order to produce anti-IL-8 antibody for use in the present invention, the antibody gene is placed into an expression vector so as to be expressed under the control of the expression regulatory region, for example an enhancer and/or a promoter. Subsequently, the expression vector is transfected into a host cell and the antibody is then expressed therein.

An antibody gene may be expressed by placing separately DNA encoding a heavy chain (H chain) or a light chain (L chain) of an antibody into a respective expression vector and co-transfecting into the host cells, or by placing DNA encoding an H chain and an L chain into a single expression vector and transfecting into host cells (International Patent Application WO 94/11523).

### 4. Altered antibody

As recombinant antibodies for use in the present invention, artificially altered recombinant antibodies such as chimeric antibody and humanized antibody can be used for the purpose of lowering heterologous antigenicity against humans. Altered antibodies can have the C region of human antibody and antibodies such as chimeric antibody or humanized antibody can be used. These altered antibody can be produced using known methods.

Chimeric antibody can be obtained by ligating the thus obtained DNA encoding the V region of non-human antibody to DNA encoding the C region of human antibody, which is then placed into an expression vector and transfected into a host for production of the antibody therein (see European Patent Application EP 125023, and International Patent Application WO 96/02576). Using this known method, chimeric antibody useful for the present invention can be obtained.

E. coli having the plasmid containing the L chain or the H chain of chimeric WS-4 antibody has been internationally deposited under the provisions of the Budapest Treaty as Escherichia coli DH5α (HEF-chWS4L-gκ) and Escherichia coli JM109 (HEF-chWS4H-gγ1) on July 12, 1994 with the National Institute of Bioscience and Human Technology, Agency of Industrial Science and Technology, of 1-3, Higashi 1-chome, Tsukuba-shi, Ibaraki, Japan, as FERM BP-4739 and FERM BP-4740, respectively.

Humanized antibody which is also called reshaped human antibody has been made by transplanting the complementarity determining region (CDRs) of non-human antibody of a mammal, for example mouse antibody, into CDRs of human antibody. The general recombinant DNA technology for preparation of such antibodies is also known (see European Patent Application EP 125023 and International Patent Application WO 96/02576).

Specifically, a DNA sequence which was designed to ligate the CDRs of mouse antibody with the framework regions (FRs) of human antibody is synthesized from several divided oligonucleotides having sections overlapping with one another at the ends thereof, and the oligonucleotides are then synthesized into one integrated DNA. The DNA thus obtained is ligated to the DNA encoding the C region of human antibody and then is placed into an expression vector, which is transfected into a host for antibody production (see European Patent Application EP 239400 and International Patent Application WO 96/02576).

The FRs of human antibody to be ligated to CDRs are selected so that the CDRs form a favorable antigen-binding site. When desired, amino acids in the FRs of antibody V region may be substituted so that the CDRs of humanized antibody may form an appropriate antigen biding site (Sato, K. et al., Cancer Res. (1993) 53, 851-856).

A preferred embodiment of humanized antibody for use in the present invention includes humanized WS-4 antibody (see International Patent Application WO 96/02576). In the humanized WS-4 antibody, CDRs of the WS-4 antibody derived from a mouse has been ligated to the FRs of the human antibody REI for the L chain, and the FR1-3 of the human antibody VDH26 and the FR4 of the human antibody 4B4 for the H chain, and part of the amino acid residues of the FR has been substituted to obtain antigen-binding activity.

E. coli having the plasmid containing the L chain or the H chain of humanized WS-4 antibody has been deposited under the provisions of the Budapest Treaty as Escherichia coli DH5α (HEF-RVLa-gκ) and Escherichia coli JM109 (HEF-RVHg-gγ1) on July 12, 1994 with the National Institute of Bioscience and Human Technology, Agency of Industrial Science and Technology, of 1-3, Higashi 1-chome, Tsukuba-shi, Ibaraki, Japan, as FERM BP-4738 and FERM BP-4741, respectively.

In order to produce anti-IL-8 antibody for use in the present invention, the antibody gene is placed into an expression vector so as to be expressed under the control of the expression regulatory region, for example an enhancer and/or a promoter. Subsequently, the expression vector is transfected into a host cell and the antibody is then expressed therein.

The antibody gene may be expressed by placed separately DNA encoding a heavy chain (H chain) or a light chain (L chain) of an antibody into a respective expression vector and co-transfecting into the host cells, or by placing DNAs encoding an H chain and an L chain into a single expression vector and transfecting the host cell (International Patent Application WO 94/11523).

Chimeric antibody consists of V regions of non-human antibody derived from a mammal and the C region derived from human antibody, whereas humanized antibody consists of CDRs of non-human antibody derived from a mammal and FRs and the C region of antibody derived from human antibody. Accordingly, since the amino acid sequences derived from a non-human mammal are reduced to a minimum in the above antibodies, antigenicity thereof in the human body is reduced so that they are useful as the active ingredient of the therapeutic agents of the present invention.

As the C region of human antibody, there can be used, for example, Cγ1, Cγ2, Cγ3, or Cγ4. The C region of human antibody may also be modified in order to improve the stability of antibody and of the production thereof. For example, when the antibody subclass IgG4 is chosen, the amino acid sequence Cys-Pro-Ser-Cys-Pro of part of the hinge region of IgG4 can be converted to the amino acid sequence Cys-Pro-Pro-Cys-Pro of the hinge region of IgG1 to resolve the structural instability of IgG4 (Angal, S. et al., Mol. Immunol. (1993) 30, 105-108).

### 5. Antibody fragments and modified antibody

Antibodies for use in the present invention may be fragments of antibody or modified versions thereof as long as they bind to IL-8 and thereby inhibit the activity of IL-8. For example, as fragments of antibody, there may be mentioned Fab, F(ab')₂, Fv or single-chain Fv (scFv) in which Fv's of H chain and L chain were ligated via a suitable linker. Specifically antibodies are treated with an enzyme, for example, papain or pepsin, to produce antibody fragments, or genes encoding these antibody fragments are constructed, and then placed into an expression vector, which is expressed in a suitable host cell (see, for example, Co, M.S. et al., J. Immunol. (1994) 152, 2968-2976; Better, M. and Horwitz, A.H., Methods Enzymol. (1989) 178, 476-496; Pluckthun, A. and Skerra, A., Methods Enzymol. (1989) 178, 497-515; Lamoyi, E., Methods Enzymol. (1986) 121, 652-663; Rousseaux, J. et al., Methods Enzymol. (1986) 121, 663-669; Bird, R.E. and Walker, B. W., Trends Biotechnol. (1991) 9, 132-137).

scFv can be obtained by ligating a V region of an H chain and a V region of an L chain of an antibody. In the scFv, the V region of H chain and the V region of L chain are preferably ligated via a linker, preferably a peptide linker (Huston, J.S. et al., Proc. Natl. Acad. Sci. U.S.A. (1988) 85, 5879-5883). The V region of H chain and the V region of L chain in the scFv may be derived from any of the above-mentioned antibodies. As the peptide linker for ligating the V regions, any single-chain peptide comprising, for example, 12 - 19 amino acid residues may be used.

DNA encoding scFv can be obtained using DNA encoding an H chain or an H chain V region of an above antibody and DNA encoding an L chain or an L chain V region of the above antibody as the template by amplifying the portion of the DNAS encoding the desired amino acid sequences among the above sequences by the PCR technique with a primer pair specifying the both ends thereof, and by further amplifying a combination of DNA encoding a peptide linker portion and the primer pair which defines that both ends of said DNA be ligated to an H chain and an L chain, respectively.

Once DNA encoding scFv is constructed, an expression vector containing it and a host transformed with said expression vector can be obtained by the conventional methods, and scFv can be obtained using the resultant host by the conventional methods.

These antibody fragments can be produced by obtaining a gene thereof in a similar manner to that mentioned above and by allowing it to be expressed in a host. "Antibody" as used in the claim of the present application encompasses these antibody fragments.

As modified antibodies, anti-IL-8 antibody conjugated with various molecules such as polyethylene glycol (PEG) can be used. "Antibody" as used in the claim of the present application encompasses these modified antibodies. These modified antibodies can be obtained by chemically modifying the antibodies thus obtained. These methods have already been established in the art.

### 6. Expression and production of recombinant antibody, altered antibody, and antibody fragment

Antibody genes constructed as mentioned above may be expressed and obtained in a known manner. In the case of mammalian cells, expression may be accomplished using an expression vector containing a commonly used useful promoter, an antibody gene to be expressed, and DNA in which the poly A signal has been operably linked at 3' downstream thereof. Examples of the promoter/enhancer include human cytomegalovirus immediate early promoter/enhancer.

Additionally, as the promoter/enhancer which can be used for expression of antibody for use in the present invention, there can be used viral promoters/enhancers such as retrovirus, polyoma virus, adenovirus, and simian virus 40 (SV40), and promoters/enhancers derived from mammalian cells such as human elongation factor 1α (HEF1α).

For example, expression may be readily accomplished by the method of Mulligan, R.C. et al. (Nature (1979) 277, 108-114) when SV40 promoter/enhancer is used, and by the method of Mizushima, S. et al. (Nucleic Acids Res. (1990) 18, 5322) when HEF1α promoter/enhancer is used.

In the case of E. coli, expression may be conducted by operably linking a conventionally used promoter, a signal sequence for antibody secretion, and an antibody gene to be expressed, followed by expression thereof. As the promoter, for example, there can be mentioned lacZ promoter and araB promoter. The method of Ward, E.S. et al. (Nature (1989) 341, 544-546; FASEB J. (1992) 6, 2422-2427) may be used when lacZ promoter is used, and the method of Better, M. et al. (Science (1988) 240, 1041-1043) may be used when araB promoter is used.

As a signal sequence for antibody secretion, when produced in the periplasm of E. coli, the pelB signal sequence (Lei, S.P. et al., J. Bacteriol. (1987) 169, 4379-4383) can be used. After separating the antibody produced in the periplasm, the structure of the antibody is appropriately refolded before use (see, for example, International Patent Application WO 96/30394).

As an origin of replication, there can be used those derived from SV40, polyoma virus, adenovirus, bovine papilloma virus (BPV), or the like. Furthermore, for amplification of the gene copy number in a host cell system, expression vectors can include as selectable markers such as the aminoglycoside transferase (APH) gene, the thymidine kinase (TK) gene, E. coli xanthine guaninephosphoribosyl transferase (Ecogpt) gene, the dihydrofolate reductase (dhfr) gene, or the like.

For the production of antibody for use in the present invention, any production system can be used, and the production system of antibody preparation comprises an in vitro or an in vivo production system.

As the in vitro production system, there can be mentioned a production system which employs eukaryotic cells and the production system which employs prokaryotic cells.

When eukaryotic cells are used, there are the production systems which employ animal cells, plant cells, and fungal cells. Known animal cells include (1) mammalian cells such as CHO cells, COS cells, myeloma cells, baby hamster kidney (BHK) cells, HeLa cells, and Vero cells, (2) amphibian cells such as Xenopus oocytes, or (3) insect cells such as sf9, sf21, and Tn5. Known plant cells include, for example, those derived from the Nicotiana family, more specifically cells derived from Nicotiana tabacum which is subjected to callus culture. Known fungal cells include (1) yeasts such as the Saccharomyces family, more specifically Saccharomyces cerevisiae, or (2) filamentous fungi such as the Aspergillus family, more specifically Aspergillus niger.

When prokaryotic cells are used, there are the production systems which employ bacterial cells. Known bacterial cells include Escherichia coli, and Bacillus subtilis.

By introducing via transformation the gene of the desired antibody into these cells and culturing the transformed cells in vitro, the antibody can be obtained. Culturing is conducted by the known methods. For example, as the culture medium for mammalian cells, DMEM, MEM, RPMI1640, IMDM and the like can be used, and serum supplements such as fetal calf serum (FCS) may be used in combination. In addition, antibodies may be produced in vivo by implanting cells into which the antibody gene has been introduced into the abdominal cavity of an animal, and the like.

As an in vivo production system, there can be mentioned those which employ animals and those which employ plants. When animals are used, there are the production systems which employ mammals and insects.

As mammals, goats, pigs, sheep, mice, and cattle can be used (Glaser, V., SPECTRUM Biotechnology Applications, 1993). When mammals are used, transgenic animals can be used. For example, antibody genes are placed into the middle of the gene encoding proteins which are inherently produced in the milk such as goat β casein to prepare fusion genes. DNA fragments containing the fusion gene into which the antibody gene has been inserted are injected to a goat embryo, and the embryo is introduced into a female goat. The desired antibody is obtained from the milk produced by the transgenic goat born to the goat who received the embryo or offsprings thereof. In order to increase the amount of milk produced containing the desired antibody produced by the transgenic goat, hormones may be given to the transgenic goat as appropriate. (Ebert, K.M. et al., Bio/Technology (1994) 12, 699-702).

As insects, silkworms can be used. When silkworms are used, baculovirus into which the desired antibody gene has been inserted is infected to the silkworm, and the desired antibody can be obtained from the body fluid of the silkworm (Maeda, S. et al., Nature (1985) 315, 592-594).

Moreover, when plants are used, tabacco, for example, may be used. When tabacco is used, the desired antibody gene is inserted into an expression vector for plants, for example pMON 530, and then the vector is introduced into a bacterium such as Agrobacterium tumefaciens. The bacterium is then infected to tobacco such as Nicotiana tabacum to obtain the desired antibody from the leaves of the tobacco (Ma, J.K. et al., Eur. J. Immunol. (1994) 24, 131-138).

Antibody gene is introduced, as mentioned above, into these animals or plants, and then the antibody is produced in such animals and plants and is recovered therefrom.

When antibody is produced in in vitro or in vivo production systems, as mentioned above, DNAs encoding an H chain or L chain of an antibody is separately placed into a respective expression vector and the host cells are co-transfected therewith, or DNAs encoding an H chain and an L chain of antibody is placed into a single expression vector and the host is transfected therewith (International Patent Application WO 94/11523).

### 7. Separation and purification of antibody

Antibodies expressed and produced as described above can be separated from inside or outside of the cell or from the host and then may be purified to homogeneity. Separation and purification of antibody for use in the present invention may be accomplished by methods of separation and purification conventionally used for proteins without any limitation. For example, separation and purification of antibody may be accomplished by combining, as appropriate, chromatography columns such as affinity chromatography, filters, ultraconcentration, salting-out, dialysis and the like (Antibodies: A Laboratory Manual, Ed Harlow and David Lane, Cold Spring Harbor Laboratory, 1988). As the column used for affinity chromatography, there can be mentioned Protein A column and Protein G column. Examples of the column employing Protein A column are Hyper D, POROS, Sepharose F.F. (Pharmacia) and the like. Chromatography other than affinity chromatography includes, for example, ion exchange chromatography, hydrophobic chromatography, gel-filtration, reverse-phase chromatography, absorption chromatography and the like (Strategies for Protein Purification and Characterization: A Laboratory Course Manual, Ed Daniel R. Marshak et al., Cold Spring Harbor Laboratory Press, 1996). Furthermore, said chromatography may be carried out using a liquid-phase chromatography such as HPLC, FPLC, and the like.

### 8. Measurement of antibody concentration

The concentration of antibody obtained as above can be determined by measurement of absorbance or by the enzyme-linked immunosorbent assay (ELISA) and the like. Thus, when absorbance measurement is employed, the antibody obtained is appropriately diluted with PBS and then the absorbance is measured at 280 nm, followed by calculation using the absorption coefficient of, though different with different species and subclasses, 1.4 OD at 1 mg/ml in the case of human antibody. When the ELISA method is used, measurement is conducted as follows. Thus, 100 µl of goat anti-human IgG antibody diluted to 1 µg/ml in 0.1 M bicarbonate buffer, pH 9.6, is added to a 96-well plate (Nunc), and is incubated overnight at 4 °C to immobilize the antibody. After blocking wells, 100 µl each of appropriately diluted antibody of the present invention or samples containing the antibody, or 100 µl of human IgG of a known concentration as the concentration standard is added, and incubated at room temperature for 1 hour. After washing wells, 100 µl of 5000-fold diluted alkaline phosphatase-labeled anti-human IgG antibody is added, and incubated at room temperature for 1 hour. After washing, the substrate solution is added and incubated, followed by measurement of absorbance at 405 nm using the MICROPLATE READER Model 3550 (Bio-Rad) to calculate the concentration of the desired antibody based on the absorbance of the concentration standard IgG.

For determination of antibody concentration, BIAcore (Pharmacia) can be used.

### 9. Confirmation of the activity of antibody

Known methods can be used for the measurement of the antigen-binding activity (Antibodies: A Laboratory Manual. Ed Harlow and David Lane, Cold Spring Harbor Laboratory, 1988) and the ligand receptor binding inhibition activity (Harada, A. et al., Int. Immunol. (1993) 5, 681-690) of the antibody used in the present invention.

As methods for determining the antigen-binding activity of anti-IL-8 antibody for use in the present invention, there can be used ELISA, EIA (enzymeimmunoassay), RIA (radioimmunoassay), or the fluorescent antibody method.

When ELISA is employed, for example, IL-8 is added to a 96-well plate onto which polyclonal antibody against IL-8 has been immobilized, and then samples containing the desired anti-IL-8 antibody, for example a culture supernatant of anti-IL-8 antibody-producing cells or purified antibody, is added thereto. Secondary antibody that recognizes the desired anti-IL-8 antibody labeled with an enzyme such as alkaline phosphatase is added, and the plate is incubated, washed, and then the enzyme substrate is added, and the absorbance is measured to evaluate the antigen-binding activity.

As methods for measuring the inhibition activity of ligand receptor binding of the anti-IL-8 antibody for use in the present invention, the conventional Cell ELISA or the ligand receptor binding assay can be used.

In the case of Cell ELISA, for example, blood cells or cancer cells expressing IL-8 receptors such as neutrophils are cultured in a 96-well plate to allow the cells to adhere thereonto, which is then immobilized with paraformaldehyde etc. Alternatively, the membrane fractions of cells expressing IL-8 receptors are prepared and 96-well plates on which the fractions have been immobilized are prepared. To this are added a sample containing the desired anti-IL-8 antibody, for example a culture supernatant of anti-IL-8 antibody-producing cells, or purified antibody, and IL-8 which is labeled with a radioisotope such as ¹²⁵I, and then the plate is incubated, washed, and radioactivity is measured to determine the amount of IL-8 bound to the IL-8 receptor and thereby to evaluate the inhibition activity of ligand receptor binding of anti-IL-8 antibody.

In the inhibition assay of IL-8 binding to IL-8 receptors on the cells, blood cells or cancer cells expressing IL-8 receptors such as neutrophils are separated by means of centrifugation etc. to prepare a cell suspension. A solution of IL-8 labeled with a radioisotope such as ¹²⁵I, or a mixture of unlabeled IL-8 and labeled IL-8, and a solution comprising anti-IL-8 antibody whose concentration has been adjusted are added to the cell suspension. After incubating for a certain period of time, the cells are separated, and the radioactivity of the labeled IL-8 bound onto the cell is measured.

As methods for measuring the neutrophil chemotaxis inhibiting ability of anti-IL-8 antibody for use in the present invention, a known method such as the one described by Grab, P.M. et al. (J. Biol. Chem. (1990) 265, 8311-8316) can be used.

Specifically, using a commercial chemotaxis chamber, anti-IL-8 antibody is diluted with a culture medium such as RPMI 1640, DMEM, MEM, or IMDM, and then IL-8 is added thereto, which is dispensed into the bottom layer of the chamber partitioned by filters. Subsequently, a prepared cell suspension, for example a neutrophil suspension, is added to the upper layer of the chamber and then allowed to stand for a certain period of time. Migrating cells will adhere to the bottom surface of the filter attached to the chamber, and therefore the number of cells adhered thereto can be measured by a method using a stain or fluorescent antibody etc. Also, visual examination under the microscope or automatic measurement using a counting device can be employed.

### 10. Method of administration and pharmaceutical preparation

Therapeutic agents that contain as an active ingredient the anti-IL-8 antibody of the present invention may be administered parenterally, for example intravenous injection such as drip infusion, intramuscular injection, intraperitoneal injection, subcutaneous injection, intratracheal injection, inhalation via a nebulizer, and the like, either systemically or locally. The method of administration may be chosen, as appropriate, depending on the age and the conditions of the patient. A therapeutic agent comprising as an active ingredient anti-IL-8 antibody of the present invention may be administered to a patient suffering from a disease in an amount sufficient to treat the disease and the symptoms of complications thereof or to prevent them at least partially.

For example, the effective dosage is chosen from the range of 0.01 mg to 1000 mg per kg of body weight per administration. Alternatively, the dosage in the range of 5 to 2000 mg per patient may be chosen. However, the dosage of a therapeutic agent comprising an anti-IL-8 antibody of the present invention is not limited to these dosages.

The timing of administration may be after the onset of acute lung injury, acute respiratory distress syndrome, or adult respiratory distress syndrome resulting from indirect causes such as the sepsis syndrome, severe nonthoracic trauma, hypertransfusion during emergency resuscitation, and an artificial cardiopulmonary bypass surgery, or administration may be conducted in a preventive manner when the onset of acute lung injury, acute respiratory distress syndrome, or adult respiratory distress syndrome resulting from the sepsis syndrome, severe nonthoracic trauma, hypertransfusion during emergency resuscitation, and an artificial cardiopulmonary bypass surgery is expected.

The period of administration may be chosen, as appropriate, depending on the age and the conditions of the patient.

Therapeutic agents that contain as the active ingredient the anti-IL-8 antibody of the present invention may be formulated into a pharmaceutical preparation according to a conventional method (Remington's Pharmaceutical Science, latest edition, Mark Publishing Company, Easton, U.S.A.), and may further contain pharmaceutically acceptable carriers or additives.

Examples of such carriers or pharmaceutical additives include water, a pharmaceutically acceptable organic solvent, collagen, polyvinyl alcohol, polyvinylpyrrolidone, a carboxyvinyl polymer, carboxymethylcellulose sodium, polyacrylic sodium, sodium alginate, water-soluble dextran, carboxymethyl starch sodium, pectin, methyl cellulose, ethyl cellulose, xanthan gum, gum Arabic, casein, agar, polyethylene glycol, diglycerin, glycerin, propylene glycol, Vaseline, paraffin, stearyl alcohol, stearic acid, human serum albumin (HSA), mannitol, sorbitol, lactose, pharmaceutically acceptable surfactants and the like.

Actual additives are chosen from, but not limited to, the above or combinations thereof depending on the dosage form of a therapeutic agent of the present invention.

When used as a parenteral injection, purified anti-IL-8 antibody may be dissolved in a solvent, for example, physiological saline, a buffer, a glucose solution, etc., to which are added an anti-adsorption agent such as Tween 80, Tween 20, gelatin, human serum albumin etc. Alternatively, a lyophilized agent which is reconstituted prior to use may be used, and as an excipient for lyophilization, sugar alcohols and sugars such as mannitol, glucose etc. can be used.

According to the present invention, acute lung injury resulting from indirect causes is the subject of treatment. Acute lung injury follows a course of acute onset and exhibits hypoxemia and bilateral diffuse infiltrative shadow in the chest x-ray radiograph, and besides these clinical findings are considered not to result from left atrial or pulmonary capillary high blood pressure. As a specific measure of hypoxemia, acute lung injury is defined by a PaO₂/FIO₂ value of 300 mmHg or lower which is an index of the respiratory function. Among acute lung injuries, those having severe hypoxemia, i.e. specifically having a PaO₂/FIO₂ value of 200 mmHg or lower, are diagnosed as acute respiratory distress syndrome or adult respiratory distress syndrome.

Causes of these diseases are broadly divided into the direct causes that occur in the lung itself and directly injure it and the indirect causes that occur systemically and indirectly injure the lung, with their mechanism of onset being greatly different from each other. The direct causes are classified into aspiration, diffuse pulmonary infection, near-drowning, inhalation of irritant gas, and lung contusion. The indirect causes are classified into the sepsis syndrome, severe nonthoracic trauma, hypertransfusion during emergency resuscitation, and an artificial cardiopulmonary bypass surgery.

Of the highest interest among the causative diseases is the sepsis syndrome, in which the cells and endotoxin are believed to act as a stimulating substance. The cells and endotoxin evoke inflammatory reactions in the blood, which, combined with secondary reactions, induce the onset of the disease.

As an experimental system for the above diseases, as mentioned in the examples below, acute lung injury was induced using the dead cell OK-432 and endotoxin, which caused hypoxemia and increased vascular permeability in the lung tissue. A therapeutic agent comprising the anti-IL-8 antibody of the present invention as an active ingredient prevented the reduction of arterial partial oxygen pressure and the increase in vascular permeability of the lung tissue, and also enhanced survival rate.

Accordingly, a therapeutic agent comprising the anti-IL-8 antibody of the present invention as an active ingredient is useful as a therapeutic agent for acute lung injury resulting from indirect causes. Furthermore, therapeutic agents comprising the anti-IL-8 antibody of the present invention as an active ingredient are useful for hypoxemia in acute lung injury resulting from indirect causes.

### Examples

The present invention will now be explained hereinbelow in more detail with reference to the following working examples, reference examples and experimental examples. It is to be noted that the present invention is not limited to these examples in any way.

### Reference example 1. Construction of a hybridoma that produces monoclonal antibody against human IL-8

Human IL-8 was given to BALB/c mice according to the conventional method, and splenocytes were collected from the mice in which immunization was established. According to the conventional method which utilizes polyethylene glycol, the splenocytes were fused with the mouse myeloma cell P3X63Ag8.653 to construct a hybridoma that produces monoclonal antibody against human IL-8. After screening using the binding activity to human IL-8 as an index, the hybridoma cell line WS-4 was obtained. Antibodies produced by the hybridoma WS-4 had the activity of inhibiting the binding of IL-8 to neutrophils, i.e. neutralizing activity (Ko, Y. et al., J. Immunol. Methods (1992) 149, 227-235).

The isotypes of the H chain and the L chain of the antibody produced by the hybridoma WS-4 were examined using the mouse monoclonal antibody isotyping kit. The result revealed that the antibody produced by the hybridoma WS-4 has mouse κ type L chain and mouse γ1 type H chain.

The hybridoma cell line WS-4 was internationally deposited under the provisions of the Budapest Treaty on April 17, 1996 with the National Institute of Bioscience and Human Technology, Agency of Industrial Science and Technology, of 1-3, Higashi 1-chome, Tsukuba-shi, Ibaraki, Japan, as FERM BP-5507.

### Reference example 2. Construction of a humanized antibody against human IL-8

The humanized WS-4 antibodies were constructed as described in International Patent Application WO 96/02576.

From the hybridoma WS-4 prepared in Reference example 1, total RNA was prepared in the conventional method, and single-stranded cDNA was synthesized therefrom. By the PCR method, DNA encoding the V regions of H chain and L chain of the mouse WS-4 antibody were amplified. The primers used in the PCR method are those described in Jones, S.T. and Bendig, M.M., Bio/Technology (1991) 9, 88-89. The PCR-amplified DNA fragments were purified, and the DNA fragment containing the gene encoding the L chain V region of the mouse WS-4 antibody and the DNA fragment containing the gene encoding the H chain V region of the mouse WS-4 antibody were isolated. These DNA fragments were ligated to a respective plasmid pUC cloning vector, which was then introduced into competent E. coli cells to obtain an E. coli transformant.

The transformant was cultured in the conventional method, and from the cell mass thus obtained plasmids containing the above DNA fragments were purified. The base sequences of DNA encoding the V regions in the plasmids were determined in the conventional method, and the CDRs of each V regions were identified from the amino acid sequence.

In order to construct vectors that express chimeric WS-4 antibody, cDNAs encoding the V regions of L chain and H chain of mouse WS-4 antibody were separately placed into respective HEF vectors that had been previously ligated to DNA encoding human C region.

In order to construct humanized WS-4 antibodies, genetic engineering technique based on the CDR grafting method was used to implant the CDR of V regions of mouse WS-4 antibody to human antibody. In order to form appropriate antigen-binding sites, substitutions of DNA sequences for partial substitution of amino acids in the FR of V region of CDR-grafted antibody were conducted.

In order to express the V regions of L chain and H chain of humanized WS-4 antibodies thus constructed as antibodies in mammalian cells, DNA encoding each was separately inserted into HEF vector, and a vector expressing the L chain or the H chain of humanized WS-4 antibody was constructed.

By cotransfection of these two expression vectors into COS cells, cell lines that produce humanized WS-4 antibodies were established. The abilities to bind to and neutralize IL-8 of the humanized WS-4 antibodies obtained by culturing the thus obtained cell lines were evaluated by ELISA and the inhibition test of IL-8/neutrophil binding, respectively. The results revealed that the humanized WS-4 antibodies inhibit the binding of IL-8 to neutrophils by binding to human IL-8 in an extent similar to that of mouse WS-4 antibody.

E. coli having the plasmid containing the L chain or the H chain of humanized WS-4 antibody was internationally deposited under the provisions of the Budapest Treaty as Escherichia coli DH5α (HEF-RVLa-gκ) and Escherichia coli JM109 (HEF-RVHg-gγ1) on July 12, 1994 with the National Institute of Bioscience and Human Technology, Agency of Industrial Science and Technology, of 1-3, Higashi 1-chome, Tsukuba-shi, Ibaraki, Japan, as FERM BP-4738 and FERM BP-4741, respectively.

### Example 1.

To Japanese white rabbits (N=8 per group) (female weighing 2.5 kg, purchased from Sankyo Lab Service) was intramuscularly given 150 mg Ketalar per rabbit and 40 mg Nembutal was injected via the ear vein to anesthetize the animals. After fixing the animals in the supine position under anesthesia on the operating table, SURFLO^{®} LV. catheter 20G (Terumo) was percutaneously punctured into the trachea of the rabbit, in which the outer catheter was allowed to dwell. To the outer catheter of SURFLO LV. catheter was connected the blast nozzle of the fork connector of Volheal adjuster set (the two-liquid mixing set, 3.5 ml, Nipro Iko).

One of the two inlets of the fork connector was connected via an extension tubing to an outlet of the MERA HFO ventilator (Senkou Ika Kogyo K.K.). Into another inlet of the fork connector was connected a 2.5 ml syringe into which 2 ml per rabbit of OK-432 suspension (Chuigai Pharmaceutical) suspended at a concentration of 2.5 KE/ml with physiological saline had been dispensed. By blowing out compressed air with the MERA HFO ventilator adjusted at a pressure of 1.0 kgt/cm² and a frequency of 12 Hz, OK-432 suspension was injected into the trachea of the rabbit. By this procedure, the OK-432 suspension was turned into a spray form and was comparatively evenly injected into the lung under pressure..

Twenty minutes prior to the administration of this OK-432, 2.5 mg of mouse WS-4 antibody against human IL-8 or 2.5 mg of mouse anti-yeast glutathione reductase antibody of the same isotype as the WS-4 antibody as the control antibody were diluted in 4 ml of physiological saline, and then each antibody was injected via the ear vein of the animal.

Thirty six hours after the administration of OK-432, lipopolysaccharide (LPS) derived from E. coli 055B5 was dissolved in phosphate buffered saline (PBS) at a concentration of 3 mg/ml, and then was injected at a dose of 1 mg/kg body weight via the ear vein. Twenty minutes prior to the LPS administration, 2.5 mg of the mouse WS-4 antibody or the mouse control antibody which is the same one as the antibody injected prior to the OK-432 administration was injected via the ear vein.

After LPS administration, one ml of the blood was successively drawn with heparin from the femoral artery. The blood was used to measure arterial partial blood oxygen pressure (PaO₂) and arterial partial carbon dioxide pressure (PaCO₂) by Radiometer Copenhagenn (model: ABL3, ACID BASE LABORATORY).

### 1) Life lengthening effect of WS-4 antibody on a model of acute lung injury resulting from indirect causes

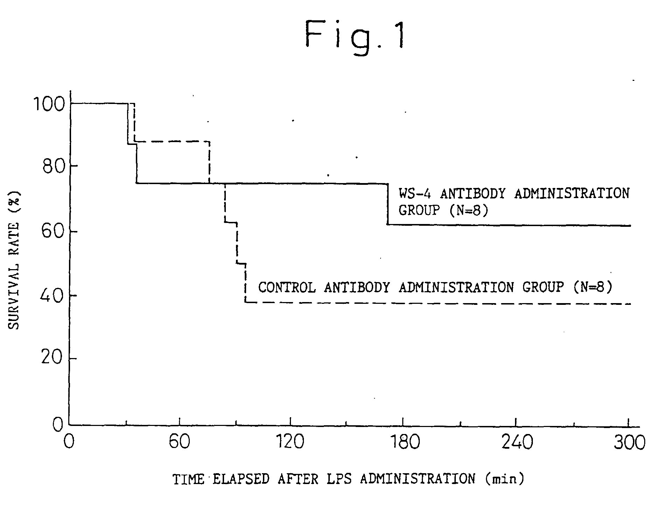

From 30 minutes after LPS administration, animals of both of the WS-4 antibody administration group and the control antibody administration group started to die. The survival rate at 5 hours later was 62.5% for the WS-4 antibody administration group and 37.5% for the control antibody administration group, indicating that the WS-4 antibody administration group has shown a statistically significant life lengthening effect as compared to the control antibody administration group. The survival curve is shown in Fig. 1.

### 2) Effect of WS-4 antibody on arterial blood gas values in a model of acute lung injury resulting from indirect causes

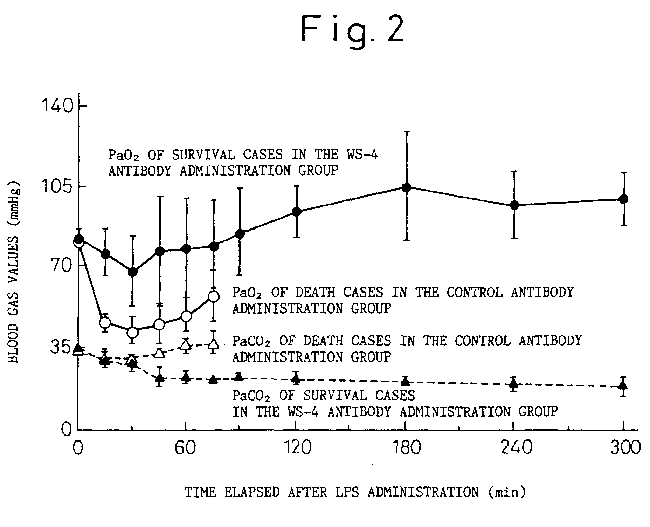

The results of blood gas values of death cases (N=5) in the control antibody administration group and of survival cases (N=5) in the WS-4 antibody administration group are shown in Fig. 2. PaO₂ of the death cases in the control antibody administration group started to decline at 15 minutes after the LPS administration and died with the value being kept low. On the other hand, PaO2 of the survival cases in the WS-4 antibody administration group maintained the normal value and survived. Thus, in the experimental system used this time, animals followed a similar course to that of acute lung injury resulting from the sepsis syndrome in that the animal who became hypoxemic after intravenous injection of LPS died.

### 3) Wet/dry weight ratio of the lung

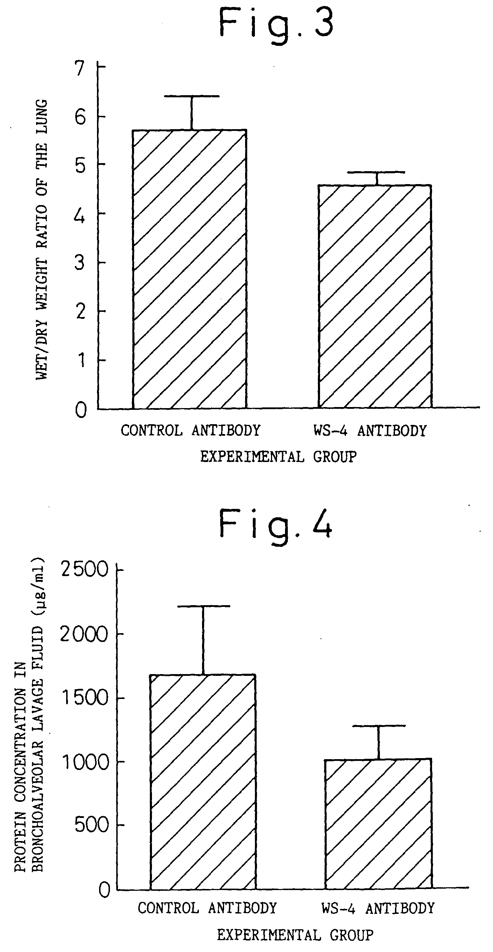

The lung was excised and the extra tissue other than the lung was removed to measure the wet weight of the lung. Then, the lung was dried at 60 °C for 24 hours to measure the dry weight. The wet/dry weight ratio was calculated by dividing the wet weight by the dry weight (Fig. 3).

The result indicated that the wet/dry weight ratio of the control antibody administration group was 5.67 ± 0.72, while that of the WS-4 antibody administration group was 4.57 ± 0.29. Since there is no difference in the dry weight of the lung between the two groups, this means that WS-4 antibody suppresses the wet weight of the lung and suppressed pulmonary edema resulting from increased vascular permeability in the lung.

### 4) Protein concentration in the bronchoalveolar lavage

After excising, the lung was washed three times with 10 ml of physiological saline and then the wash solution was recovered and centrifuged at 1500 x g for 15 minutes. The supernatant after centrifugation was collected and its protein concentration was measured with bovine serum albumin as the standard using a determination kit (PIERCE) (Fig. 4).

As a result, the protein concentration in the control antibody administration group was 1670 ± 551 µg/ml, while that in the WS-4 antibody administration group was 1010 ± 284 µg/ml. This means that WS-4 antibody suppressed protein leakage resulting from increased vascular permeability in the lung.

As hereinabove described, WS-4 antibody exhibited a life lengthening effect and a suppressive effect on vascular permeability in the lung, and hence it was demonstrated that WS-4 antibody is an therapeutic agent effective for acute lung injury resulting from indirect causes such as the sepsis syndrome.

### Industrial applicability

The administration of anti-IL-8 antibody prevented endotoxin-induced acute lung injury. This fact indicates that anti-IL-8 antibody is effective as a therapeutic agent for acute lung injury resulting from indirect causes and a therapeutic agent for hypoxemia in acute lung injury resulting from indirect causes.
The name and address of the reference depository organization of microorganisms deposited under Patent Cooperation Treaty Rule 13(2):
National Institute of Bioscience and Human Technology, Agency of Industrial Science and Technology 1-3, Higashi 1-chome, Tsukuba-shi, Ibaraki, Japan

| Accession No. | Date deposited |
|---|---|
| FERM BP-4738 | July 12, 1994 |
| FERM BP-4739 | July 12, 1994 |
| FERM BP-4740 | July 12, 1994 |
| FERM BP-4741 | July 12, 1994 |
| FERM BP-5507 | April 17, 1996 |

## Claims

1. A therapeutic agent for treatment of acute lung injury resulting from indirect causes comprising anti-IL-8 antibody as an active ingredient.

2. The therapeutic agent according to claim 1 in which the acute lung injury is acute respiratory distress syndrome.

3. The therapeutic agent according to claim 1 in which the acute lung injury is adult respiratory distress syndrome.

4. The therapeutic agent according to any of claims 1, 2, and 3, in which the indirect cause is the sepsis syndrome.

5. The therapeutic agent according to any of claims 1, 2, and 3, in which the indirect cause is severe nonthoracic trauma.

6. The therapeutic agent according to any of claims 1, 2, and 3, in which the indirect cause is hypertransfusion during emergency resuscitation.

7. The therapeutic agent according to any of claims 1, 2, and 3, in which the indirect cause is artificial cardiopulmonary bypass surgery.

8. The therapeutic agent according to any of claims 1 through 7, in which the anti-IL-8 antibody is a monoclonal antibody.

9. The therapeutic agent according to any of claims 1 through 8, in which the anti-IL-8 antibody is an antibody against mammalian IL-8.

10. The therapeutic agent according to any of claims 1 through 9, in which the anti-IL-8 antibody is an antibody against human IL-8.

11. The therapeutic agent according to any of claims 1 through 10, in which the anti-IL-8 antibody is WS-4 antibody.

12. The therapeutic agent according to any of claims 1 through 11, in which the anti-IL-8 antibody has the constant region of human antibody.

13. The therapeutic agent according to any of claims 1 through 12, in which the anti-IL-8 antibody is a humanized or chimeric antibody.

14. The therapeutic agent according to any of claims 1 through 13, in which the anti-IL-8 antibody is a humanized WS-4 antibody.

15. A therapeutic agent for hypoxemia in acute lung injury resulting from indirect causes comprising anti-IL-8 antibody as an active ingredient.

16. Use of anti-IL-8 antibody for production of a therapeutic agent for treatment of acute lung injury resulting from indirect causes.

17. Use according to claim 16 in which the acute lung injury is acute respiratory distress syndrome.

18. Use according to claim 16 in which the acute lung injury is adult respiratory distress syndrome.

19. Use according to any of claims 16, 17, and 18, in which the indirect cause is the sepsis syndrome.

20. Use according to any of claims 16, 17, and 18, in which the indirect cause is severe nonthoracic trauma.

21. Use according to any of claims 16, 17, and 18, in which the indirect cause is hypertransfusion during emergency resuscitation.

22. Use according to any of claims 16, 17, and 18, in which the indirect cause is an artificial cardiopulmonary bypass surgery.

23. Use according to any of claims 16 through 22, in which the anti-IL-8 antibody is a monoclonal antibody.

24. Use according to any of claims 16 through 23, in which the anti-IL-8 antibody is an antibody against mammalian IL-8.

25. Use according to any of claims 16 through 24, in which the anti-IL-8 antibody is an antibody against human IL-8.

26. Use according to any of claims 16 through 25, in which the anti-IL-8 antibody is WS-4 antibody.

27. Use according to any of claims 16 through 26, in which the anti-IL-8 antibody has the constant region of human antibody.

28. Use according to any of claims 16 through 27, in which the anti-IL-8 antibody is a humanized or chimeric antibody.

29. Use according to any of claims 16 through 28, in which the anti-IL-8 antibody is a humanized WS-4 antibody.

30. Use of anti-IL-8 antibody for production of a therapeutic agent for hypoxemia in acute lung injury resulting from indirect causes.

31. A therapeutic method for treatment of acute lung injury resulting from indirect causes, which method comprises administering anti-IL-8 antibody to a subject in need of said therapy.

32. The method according to claim 31 in which the acute lung injury is acute respiratory distress syndrome.

33. The method according to claim 31 in which the acute lung injury is adult respiratory distress syndrome.

34. The method according to any of claims 31, 32, and 33, in which the indirect cause is the sepsis syndrome.

35. The method according to any of claims 31, 32, and 33, in which the indirect cause is severe nonthoracic trauma.

36. The method according to any of claims 31, 32, and 33, in which the indirect cause is hypertransfusion during emergency resuscitation.

37. The method according to any of claims 31, 32, and 33, in which the indirect cause is an artificial cardiopulmonary bypass surgery.

38. The method according to any of claims 31 through 37, in which the anti-IL-8 antibody is a monoclonal antibody.

39. The method according to any of claims 31 through 38, in which the anti-IL-8 antibody is an antibody against mammalian IL-8.

40. The method according to any of claims 31 through 39, in which the anti-IL-8 antibody is an antibody against human IL-8.

41. The method according to any of claims 31 through 40, in which the anti-IL-8 antibody is WS-4 antibody.

42. The method according to any of claims 31 through 41, in which the anti-IL-8 antibody has the constant region of human antibody.

43. The method according to any of claims 31 through 42, in which the anti-IL-8 antibody is a humanized or chimeric antibody.

44. The method according to any of claims 31 through 43, in which the anti-IL-8 antibody is a humanized WS-4 antibody.

45. Use of anti-IL-8 antibody for production of a therapeutic agent for hypoxemia in acute lung injury resulting from indirect causes.
